# EUROPÄISCHE PATENTSCHRIFT

(11) **EP 2 848 097 B1**
(45) Veröffentlichungstag und Bekanntmachung des Hinweises auf die Patenterteilung: **11.08.2021**
(21) Anmeldenummer: 13727064.1
(22) Anmeldetag: 08.05.2013
(51) Int. Cl.: H05H 1/24, A61B 18/04

(54) **VORRICHTUNG ZUR PLASMABEHANDLUNG VON MENSCHLICHEN, TIERISCHEN ODER PFLANZLICHEN OBERFLÄCHEN, INSBESONDERE VON HAUT ODER SCHLEIMHAUTAREALEN**
DEVICE FOR THE PLASMA TREATMENT OF HUMAN, ANIMAL OR PLANT SURFACES, IN PARTICULAR OF SKIN OR MUCOUS MEMBRANE AREAS
DISPOSITIF POUR TRAITER AU PLASMA DES SURFACES HUMAINES, ANIMALES OU VÉGÉTALES, EN PARTICULIER LA PEAU OU DES ZONES DE MUQUEUSE

(30) Priorität: 09.05.2012 DE 102012207750
(43) Veröffentlichungstag der Anmeldung: 18.03.2015
(73) Patentinhaber: Leibniz-Institut für Plasmaforschung und Technologie e.V., 17489 Greifswald (DE)
(72) Erfinder: WELTMANN, Klaus-Dieter, 18609 Binz (DE); BRANDENBURG, Ronny, 17495 Gross Kiesow (DE); KRAFCZYK, Stephan, 17489 Greifswald (DE); STIEBER, Manfred, 17489 Greifswald (DE); VON WOEDTKE, Thomas, 18519 Sundhagen (DE)
(74) Vertreter: Schulz Junghans Patentanwälte PartGmbB
(86) Internationale Anmeldenummer: PCT/EP2013/059656
(87) Internationale Veröffentlichungsnummer: WO 2013/167693

(56) Entgegenhaltungen:
- WO-A1-2011/023478
- DE-A1-102008 045 830
- JP-A- 2012 061 393
- US-A1- 2011 116 967
- US-B1- 6 494 881

## Beschreibung

Die Erfindung betrifft eine Vorrichtung zur Behandlung von Freiformflächen und Bereichen menschlicher oder tierischer Hautareale sowie pflanzlicher Oberflächen mittels eines kalten Atmosphärendruckplasmas.

### [Stand der Technik]

In der Literatur zum neuen Fachgebiet "Plasmamedizin" werden derzeit zahlreiche Untersuchungen zur Anwendbarkeit von Atmosphärendruckplasmen für medizinischtherapeutische Zwecke beschrieben (Beispielsweise: Fridman G. et al. "Sterilization of Living Human and Animal Tissue by Non-Thermal Atmospheric Pressure Dielectric Barrier Discharge Plasma" ISPC 18. Kyoto 2007; Fridman G. et al. "Blood Coagulation and Living Tissue Sterilization by Floating-Electrode Dielectric Barrier Discharge in Air", Plasma Chemistry and Plasma Processing 2006, 26, 425-442; Fridman G. et al. "Applied Plasma Medicine", Plasma Process. Polym. 2008, 5, 503-533).

Um innovative Methoden dieser Art entwickeln und anwenden zu können, ist es erforderlich, spezielle Atmosphärendruck-Plasmaquellen zu entwickeln, die eine Reihe von Bedingungen erfüllen müssen. Soll eine derartige Plasmaquelle beispielsweise im Sinne eines medizinisch-therapeutischen Pflasters eingesetzt werden, so sind folgende Voraussetzungen zu erfüllen:
(1) Die Quellen sollten zur definierten, reproduzierbaren, flächigen Behandlung beliebig gekrümmter Haut- oder Schleimhautareale oder pflanzlicher Oberflächen geeignet sein.
(2) Die Quellen sollten aus flexiblen Materialien gefertigt sein, die autoklavierbar, chemisch beständig und plasmabeständig sind.
(3) Die verwendeten Werkstoffe sollten biokompatibel sein.
(4) Die Quellen sollten eine schmerzfreie Behandlung ermöglichen, die das zu behandelnde Gewebe weder durch Temperaturbelastung, Austrocknung und die Wirkung elektrischer Felder noch durch die Wirkung der durch das Plasma erzeugten reaktiven Spezies schädigt.
(5) Es sollte die Möglichkeit der dosierten Zuführung eines speziellen Prozessgasgemisches, gegebenenfalls unter Zumischung weiterer Substanzen, sowohl unter den Bedingungen einer permanenten Gasströmung, als auch in einem abgeschlossenen Volumen, bestehen.
(6) Optional sollte die Elektrodenanordnung der Plasmaquelle derart strukturiert sein, dass sie gasdurchlässig ist und somit ein Gasfluss auf der zu behandelnden Oberfläche durch die Elektrodenanordnung hindurch erzeugt werden kann.
(7) Die Quellen sollten einfach konstruiert sein, so dass eine möglichst einfache, kostengünstige industrielle Produktion möglich ist.

Hitzebeständige, biokompatible und chemisch beständige Kunststoffe sind dem Fachmann bekannt.

Die in den Patentschriften DE3618412, WO2004/105810, WO2006/116252 beschriebenen Vorrichtungen zur Plasmabehandlung von lebendem Gewebe mit nichtthermischen Atmosphärenplasmen sind entweder mit starren Elektrodensystemen (-DBD-Elektroden) zur lokalen Behandlung kleiner Flächen oder mit "Plasma-Düsen" (-Plasmajet) zur Erzeugung von Atmosphärendruckplasmen im Bereich der zu behandelnden Gewebeoberflächen ausgerüstet. Ihr Einsatz für die kurzzeitige, definierte und reproduzierbare Behandlung größerer Hautbereiche ist daher als problematisch zu betrachten.

Als eine geeignete Möglichkeit zur flächigen Behandlung von biologischem Gewebe mittels eines kalten Atmosphärendruckplasmas wurde in einer früheren Patentschrift der Erfinder (WO2011/023478 A1) eine Vorrichtung mit einer speziellen, elastischen Elektrodenanordnung, zur Erzeugung eines flächigen Plasmas mittels einer dielektrisch behinderten Oberflächenentladung beschrieben, die sich flexibel auf beliebig gekrümmte Oberflächen auflegen lässt und somit prinzipiell für die Plasmabehandlung erkrankter Hautareale geeignet ist. Nachteilig ist in diesem Falle die Tatsache, dass mit dieser Art von Atmosphärendruck-Plasmaquellen einige der oben genannten Voraussetzungen (insbesondere die Voraussetzungen (2), (6) und (7)) nicht erfüllt sind.

Zum Stand der Technik gehört auch die Druckschrift DE 102008045830 A1 (CINOGY GmbH), auch veröffentlicht als US 2012107896 A1 oder WO 2010/025904 A2. Darin wird offenbart, dass eine Vorrichtung zur Behandlung von Oberflächen verwendet wird, die mittels einer Elektrode über ein Feststoffdielektrikum durch eine dielektrisch behinderte Gasentladung ein Plasma erzeugt, wobei die Vorrichtung eine reversibel formveränderbare Wirk-Oberfläche aufweist, die während der Behandlung unmittelbar an das Plasma angrenzt. In der Beschreibung wird im Absatz [0024] erwähnt: "... dass das Dielektrikum als Granulat und/oder als Pulver ausgestaltet ist. Dies kann jedoch auch dadurch realisiert werden, dass das Dielektrikum, beispielsweise als feines Pulver, an und/oder in einer flexiblen Hohlfaser, beispielsweise aus Gläsern, Keramiken oder Kunststoffen angeordnet ist oder das Dielektrikum selbst eine flexible Hohlfaser darstellt." Im Absatz [0068] wird weiter ausgeführt, dass "mehrere Hohlfasern aus dielektrischem Material oder mit einer dielektrischen Beschichtung aus Glas, Keramik oder Kunststoff und mit Elektroden versehen, beispielsweise in Form einer inneren Beschichtung im Verbund mit weiteren Stützelementen in Form von Fasern ein gewebeartiges Element bilden, so dass auch bei schwierigen Topologien eine entsprechend adäquate und angepasste Formung und somit Applikation möglich ist." Eine Spannungsfestigkeit des Dielektrikums von mindestens 2 kV wird dort aber nicht beschrieben. Auch wird kein Polyimid, Silikon oder Aramid offenbart.

In der europäischen Patentschrift EP 1 027 020 B1 wird beschrieben, dass mit einem kompakten stabförmigen (nicht flächigen) bipolaren Elektrodensystem in einer leitfähigen flüssigen Umgebung (Elektrolyt, z.B. NaCl-Lösung) ein Plasma erzeugt wird, das stark genug ist, einen Gewebeabtrag zu realisieren. Dort wird zwar eine "Durchschlagspannung" von mindestens 2kV genannt, aber die gesamte Anordnung ist darauf gerichtet, dass in Gegenwart eines elektrisch leitfähigen Fluids gearbeitet wird.

Abschließend seien hier noch kurz die Druckschriften DE 10 2009 002 278 A1 und EP 1158919 B1 erwähnt. DE 10 2009 002 278 A1 beschreibt eine bereits 150-Jahre alte DBD-Anordnung neben anderen bekannten Prinzipien mit einer Ultraschallquelle. In EP 1158919 B1 wird ein Verfahren zur Behandlung von Haut bzw. Gewebe offenbart.

WO2011/023478 A1 offenbart eine Vorrichtung zu Behandlung von Haut mit einem nicht-thermischen Plasma basierend auf einer dielektrisch behinderten Entladung, bei der eine flächige Hochspannungselektrode zwischen zwei Dielektrikumsschichten eingebettet ist. Eine flächige geerdete Elektrode ist auf der zu behandelnden Seite der Vorrichtung angeordnet und von der Hochspannungselektrode isoliert. Zur Herstellung der Vorrichtung, muss die Hochspannungselektrode zwischen zwei Dielektrikumsschichten sicher eingebettet werden, was die Produktion der Vorrichtung vergleichsweise kostspielig macht.

US 2011/0116967 A1 offenbart eine plasmaerzeugende Vorrichtung zum sterilisieren von medizinischen Geräten mittels einer dielektrisch behinderten Entladung.

DE 10 2008 045830 A1 lehrt eine Vorrichtung zur Behandlung von biologischem Material mit einem nicht-thermischen Plasma, das mittels einer dielektrisch behinderten Entladung erzeugt wird. Die Vorrichtung weist dazu Elektroden in Form von Hohlfasern auf.

JP 2012 061393 A offenbart die Verwendung von Polyimiden als Dielektrikum für die Hochspannungselektrode bei Vorrichtungen zur Erzeugung einer dielektrisch behinderten Entladung.

### [Aufgabe der Erfindung]

Der Erfindung liegt die Aufgabe zugrunde, eine technische Lösung für den Aufbau einer Plasmaquelle zu finden, die die oben genannten Voraussetzungen erfüllt.

### [Lösung der Aufgabe]

Die Aufgabe wird durch die Merkmale der Patentansprüche gelöst.

### [Darstellung der Erfindung]

Erfindungsgemäß wird eine spezielle Elektrodenanordnung verwendet, bei der die Hochspannungselektrode aus einem flexiblen Draht besteht, der mit einer dehnbaren Isolationsschicht ummantelt ist, und zwar so, dass die Hochspannungselektrode aus einem Isolierdraht ist, wobei die Isolationsschicht als Dielektrikum für die zu erzeugende dielektrisch behinderte Entladung dient, die sich gleichzeitig den Konturen der zu behandelnden Körperareale oder Oberflächen anpasst und eine Spannungsfestigkeit von mindestens 2 kV aufweist, wobei die Hochspannungselektrode auf einem als geerdete Elektrode (2) dienenden elektrisch leitenden textilen Material angeordnet ist. Die Isolationsschicht hat eine hohe elektrische Spannungsfestigkeit bzw. Durchschlagsspannung, weist eine für die Autoklavierbarkeit ausreichende Temperaturstabilität auf, ist chemisch beständig, biokompatibel und plasmabeständig. Isolierstoffe mit diesen speziellen Eigenschaften sind auf einige wenige Kunststoffarten beschränkt. Beispiele dafür sind in der folgenden Tabelle dargestellt:

**Tabelle 1: Eigenschaften von Materialien mit hoher Spannungsfestigkeit**

| **Kunststoffe** | **Dielektrizitätszahl** | **Spannungsfestigkeit [kV/mm]** | **Max. Temperatur [°C]** |
|---|---|---|---|
| Silikone (z.B. Silastic®, Elastosil®) | 2-3 | bis 40 | 150 - 350 (hochhitzebe-ständiges Silikon) |
| Polyimide (z.B. Kapton®-CR: (dauerhaft plasmabeständig) | 3,4 | bis 45 | 220-260 (kurz: 400) |
| Aramide (z.B. Kevlar®, Nomex®) | 3,2 | 18 bis 40 | 220 |

Ein wesentliches Merkmal für die Eignung der Isolierung ist dabei die als "plasmabeständig" bezeichnete Eigenschaft. Während bei den meisten Isoliermaterialien durch die im Plasma erzeugten reaktiven Spezies die Materialeigenschaften derart beeinflusst werden, dass die Isolation bei Dauergebrauch spröde wird und dadurch die Spannungsfestigkeit beeinträchtigt wird, bleiben die Materialeigenschaften beispielsweise von Kapton stabil. Für die erfindungsgemäße Vorrichtung bedeutet dies, dass Polyimide, vorzugsweise aromatische Polyimide, z.B. bekannt als Kapton^{®} , chemisch Poly(4,4'-oxydiphenylen-pyromellitimid), die am besten geeigneten Materialien für die Isolierung der dünnen leitenden Fäden oder des Drahtes sind, insbesondere geeignet für eine längere Behandlung der Flächen. Für eine kürzere Nutzungsdauer eignen sich auch Kunststoffe wie Silikon, Aramide (aromatische Polyamide) oder fluorpolymere Werkstoffe wie FEP, PFA, ETFE, PTFE, insbesondere Teflon®, deren Materialeigenschaften unter Plasmabedingungen zwar zeitlich begrenzt als stabil betrachtet werden können, aber nicht dauerhaft stabil sind. Die entscheidende elektrische Größe der für die Hochspannungselektrode genutzten Isolationsschicht ist die Durchschlagsspannung, die einerseits durch die Materialkenngröße der Spannungsfestigkeit (kV/mm) und andererseits durch die Dicke der Isolationsschicht bestimmt wird. Die Durchschlagsspannung der Hochspannungselektrode beträgt erfindungsgemäß mindestens 2 kV, vorzugsweise 5-10 kV.

Gute Ergebnisse werden mit Polyimiden wie Polybismaleinimid (PBMI), Polybenzimidazol (PBI), Polyoxadiazobenzimidazol (PBO), Polyimidsulfon (PISO) und Polymethacrylimid (PMI) erreicht. Bevorzugt sind aromatische Polyimide wie Poly(4,4'-oxydiphenylen-pyromellitimid). Die besten Ergebnisse werden erzielt, wenn das aromatische Polyimid zusätzlich einen anorganischen Füllstoff enthält. Ein solches Material ist unter dem Markennamen "Kapton® CR" der Firma DuPont bekannt. Anorganische Füllstoffe in elektrischen Feststoff-Isolatoren auf der Basis einer Duroplastmatrix sind aus der Offenlegungsschrift EP 1 300 439 A1 bekannt. Dazu zählen u.a. Siliziumdioxid, Aluminiumoxid, Titandioxid oder Dolomit (CaMg[CO₃]₂). Auch die Verwendung von ATH (Aluminium-Trihydrat) ist bekannt.

Erfindungsgemäß können auch Mischpolymere aus Polyimiden, Silikon, Fluorpolymeren oder Aramiden verwendet werden. Erfindungsgemäß ist es auch möglich, den Draht oder die Fäden mit verschiedenen Polymeren zu beschichten, z.B. eine Grundbeschichtung mit Polyamiden und eine weitere Beschichtung mit Silikon oder umgekehrt, je nach gewünschter Flexibilität.

Im Unterscheid zu DE 102008045830 A1 stellt die Plasmaanordnung eine sich anschmiegende Struktur dar. Die Form der Wirkfläche ändert sich nicht, denn das Dielektrikum wird nicht verformt. Auch wird kein zusätzliches Stützelement benötigt, sondern das System ist bereits stabil genug. Ein weiterer Unterschied ist die textile Variante der Plasmaquelle.

Die geerdete Elektrode zur Erzeugung einer dielektrisch behinderten Oberflächenentladung muss nicht unbedingt gasdurchlässig sein. Für bestimmte Anwendungen, z.B. Therapien, bei denen extra luftdicht abgeschottet wird - ist ein Mikroklima nötig. Im Fall der Verwendung einer gasdurchlässigen Elektrode kann das auch dadurch erreicht werden, dass eine Abdichtung gegen die äußere Atmosphäre durch eine zusätzlich aufgebrachte gasundurchlässige Schicht oder Folie erfolgt oder durch ein gasdicht abgeschlossenes Gehäuse, das erforderlichenfalls an eine verschließbare Gasleitung angeschlossen werden kann.

Gemäß einer bevorzugten Ausführungsform der Erfindung wird aber eine gasdurchlässige geerdete Elektrode zur Erzeugung einer dielektrisch behinderten Oberflächenentladung aus elektrisch leitendem, textilem Material, verwendet.

Kernstück der erfindungsgemäßen Ausführungsform ist eine spezielle, vorzugsweise gasdurchlässige, Elektrodenanordnung zur Erzeugung einer dielektrisch behinderten Oberflächenentladung, bei der vorzugsweise die geerdete Elektrode aus elektrisch leitendem, textilem Material und die Hochspannungselektrode aus einem dünnen Draht oder elektrisch leitenden Faden besteht, der mit einer Isolationsschicht ummantelt ist, die speziellen Anforderungen genügen muss. Auf der Grundlage dieser Erfindung ist es möglich, im Bereich erkrankter Hautpartien des menschlichen Körpers, in unmittelbarer Nähe der Hautoberfläche oder von Wunden ein flächiges Plasma zur Behandlung der erkrankten Areale zu erzeugen, das hinsichtlich der Belastung der Haut durch Temperatur und elektrische Spannungen unbedenklich ist. Der Vorteil der gasdurchlässigen, textilen Flächenstruktur besteht vor allem darin, dass sich die Anordnung flexibel auf beliebig gekrümmte Oberflächen auflegen lässt und darüber hinaus die Möglichkeit eines Gasaustausches mit der Umgebung bzw. der gezielten Dosierung eines speziellen Prozessgasgemisches durch das textile Material hindurch in den aktiven Plasmabereich bietet.

Auch Pflanzen (Bäume) haben Wunden. Insbesondere bei Edelgewächsen ergeben sich mit der erfindungsgemäßen Vorrichtung entsprechende Anwendungsverfahren.

### [Beispiele]

Die Erfindung wird anhand von Ausführungsbeispielen näher erläutert.

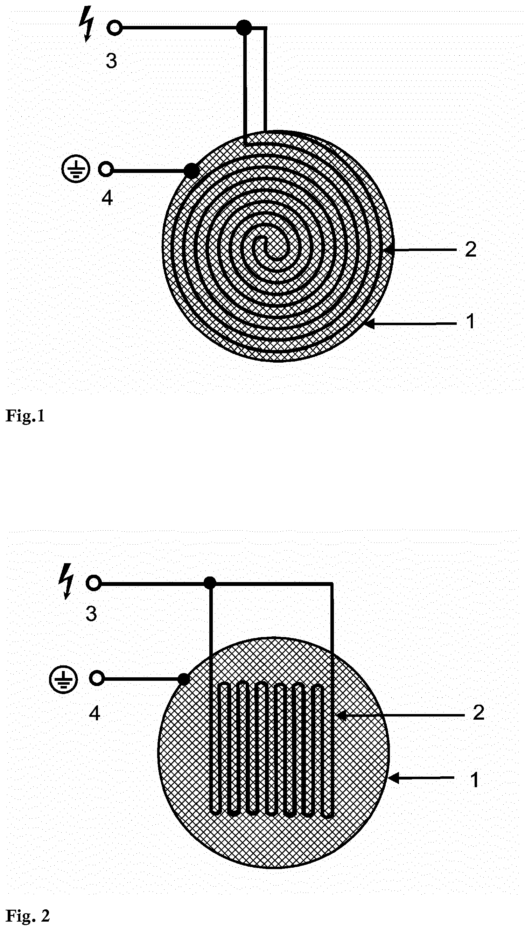

Fig. 1 und Fig. 2 zeigen hierzu schematische Darstellungen von Funktionsmustern der erfindungsgemäßen Vorrichtung. Der Unterschied beider Beispiele besteht dabei in der Anordnung der Hochspannungselektrode aus isoliertem Draht auf dem als geerdete Elektrode dienenden elektrisch leitenden textilen Material. In Fig. 1 ist die Hochspannungselektrode spiralförmig angeordnet, während in Fig. 2 eine mäanderförmige Anordnung der Hochspannungselektrode dargestellt ist.

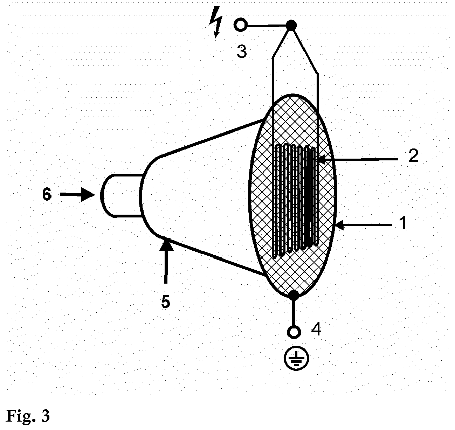

Fig. 3 zeigt den prinzipiellen Aufbau einer Variante der erfindungsgemäßen Vorrichtung, bei der die Möglichkeit einer dosierten Zuführung eines speziellen Prozessgasgemisches, gegebenenfalls unter Zumischung weiterer Substanzen, durch die gasdurchlässige Elektrodenanordnung erfolgen kann.

### [Bezugszeichenliste]

Für die nachfolgenden Zcichnungcn werden folgende Bczugszcichcn verwendet:
- 1: geerdete Elektrode aus leitfähigem gewebtem Material (z.B. aus leitfähigem textilen Material)
- 2: Hochspannungs-Elektrode aus Isolierdraht mit Isolation aus hitze- und plasmabeständigem sowie chemisch beständigem Isoliermaterial mit hoher elektrischer Spannungsfestigkeit (z.B. Kapton®-CR)
- 3: Hochspannungskabel
- 4: Massekabel
- 5: Gasdüse
- 6: Gaszufuhr

## Patentansprüche

1. Vorrichtung zur Behandlung von Bereichen menschlicher oder tierischer oder pflanzlicher Oberflächen mittels eines kalten Atmosphärendruckplasmas durch Erzeugung einer dielektrisch behinderten Oberflächenentladung, umfassend
- mindestens ein Hochspannungskabel (3),
- mindestens ein Massekabel (4),
- mindestens eine Plasmaquelle,
- mindestens eine geerdete Elektrode (1) und
- mindestens eine Hochspannungselektrode (2),
**dadurch gekennzeichnet dass**, die Hochspannungselektrode (2) aus einem
flexiblen Draht besteht, der mit einer dehnbaren Isolationsschicht ummantelt ist, und zwar so, dass die Hochspannungselektrode (2) aus einem Isolierdraht ist,
wobei die Isolationsschicht als Dielektrikum für die zu erzeugende dielektrisch behinderte Entladung dient, die sich gleichzeitig den Konturen der zu behandelnden Körperareale oder Oberflächen anpasst und eine Spannungsfestigkeit von mindestens 2 kV aufweist, wobei die Hochspannungselektrode (2) auf einem als geerdete Elektrode (1) dienenden
elektrisch leitenden textilen Material angeordnet ist.

2. Vorrichtung nach Anspruch 1, **dadurch gekennzeichnet, dass** als Isolationsschicht für die Hochspannungselektrode (2) aromatische Polyimide verwendet werden.

3. Vorrichtung nach Anspruch 2, **dadurch gekennzeichnet, dass** als Isolationsschicht für die Hochspannungselektrode (2) Poly(4,4'-oxydiphenylen-pyromellitimid) verwendet wird.

4. Vorrichtung nach Anspruch 2 oder 3, **dadurch gekennzeichnet, dass** das aromatische Polyimid anorganische Füllstoffe enthält.

5. Vorrichtung nach Anspruch 3, **dadurch gekennzeichnet, dass** als Isolationsschicht Kapton® -CR verwendet wird.

6. Vorrichtung nach Anspruch 1, **dadurch gekennzeichnet, dass** das als geerdete Elektrode (1) fungierende Material gasdurchlässig und flexibel ist.

7. Vorrichtung nach Anspruch 6, **dadurch gekennzeichnet, dass** als geerdete Elektrode (1) eine leitfähige textile Flächenstruktur fungiert, wobei die textile Flächenstruktur durch die üblichen Verfahren der Herstellung textiler Materialien gefertigt ist.

8. Vorrichtung nach Anspruch 7, **dadurch gekennzeichnet, dass** die leitfähige textile Flächenstruktur gewebt, gewirkt, geflochten oder gestrickt ist.

9. Vorrichtung nach Anspruch 1, **dadurch gekennzeichnet, dass** ein leitfähiges, gasundurchlässiges und flexibles Material als geerdete Elektrode (1) fungiert.

10. Vorrichtung nach einem der Ansprüche 1 bis 9, **dadurch gekennzeichnet, dass** sie eine Gaszufuhr (6) aufweist.

11. Vorrichtung nach Anspruch 10, **dadurch gekennzeichnet, dass** sie mindestens eine Gasdüse (5) aufweist.

12. Vorrichtung nach einem der Ansprüche 10 oder 11, **dadurch gekennzeichnet, dass** die Gaszufuhr (6) durch eine Absperrvorrichtung unterbrochen werden kann, so dass eine Behandlung in einem abgeschlossenen Volumen unter den Bedingungen eines Mikroklimas über der zu behandelnden Oberfläche erfolgen kann.

13. Vorrichtung nach einem der Ansprüche 1 bis 11, **dadurch gekennzeichnet, dass** die Elektrodenanordnung der Plasmaquelle gasdurchlässig ist und dadurch ein Gasfluss auf der zu behandelnden Oberfläche durch die Elektrodenanordnung hindurch erzeugt werden kann.

14. Pflaster, Manschette oder Wundabdeckung zum Aufbringen auf Hautbereiche, das die Vorrichtung gemäß mindestens einem der Ansprüche 1 bis 13 aufweist.

## Claims

1. A device for treating regions of human or animal or plant surfaces using a cold atmospheric-pressure plasma by generating a dielectrically impeded surface discharge, comprising
- at least one high-voltage cable (3),
- at least one ground cable (4),
- at least one plasma source,
- at least one grounded electrode (1), and
- at least one high-voltage electrode (2),
**characterized in that**
the high-voltage electrode (1) consists of a flexible wire that is coated with a stretchable insulation layer, such that the high-voltage electrode (1) consists of an insulated wire, wherein the insulation layer serves as a dielectric for the dielectrically impeded discharge to be generated, simultaneously adapts to the contours of the body areas or surfaces to be treated and has an electric strength of at least 2 kV, wherein the high-voltage electrode (1) is arranged on an electrically conductive textile material that serves as the grounded electrode (2).

2. The device according to claim 1, **characterized in that** aromatic polyimides are used as the insulation layer for the high-voltage electrode (2).

3. The device according to claim 2, **characterized in that** poly(4,4'-oxydiphenylene-pyromellitimide) is used as the insulation layer for the high-voltage electrode (2).

4. The device according to claim 2 or 3, **characterized in that** the aromatic polyimide contains inorganic fillers.

5. The device according to claim 3, **characterized in that** Kapton® CR is used as the insulation layer.

6. The device according to claim 1, **characterized in that** the material used as the grounded electrode (2) is permeable to gas and flexible.

7. The device according to claim 6, **characterized in that** a conductive textile planar structure is used as the grounded electrode (1), wherein the textile planar structure is manufactured using the conventional methods for producing textile materials.

8. The device according to claim 7, **characterized in that** the conductive textile planar structure is woven, warp-knitted, braided or weft-knitted.

9. The device according to claim 1, **characterized in that** a conductive, flexible material that is impermeable to gas is used as the grounded electrode (1).

10. The device according to any one of claims 1 to 9, **characterized in that** it comprises a gas supply (6).

11. The device according to claim 10, **characterized in that** it comprises at least one gas nozzle (5).

12. The device according to any one of claims 10 or 11, **characterized in that** the gas supply (6) can be interrupted by means of a shut-off device, such that treatment can take place in an enclosed volume under the conditions of a microclimate over the surface to be treated.

13. The device according to any one of claims 1 to 11, **characterized in that** the electrode arrangement of the plasma source is permeable to gas and, as a result, a flow of gas can be generated through the electrode arrangement on the surface to be treated.

14. An adhesive bandage, cuff or wound covering for application to regions of skin, comprising the device according to at least one of claims 1 to 13.

## Revendications

1. Dispositif pour le traitement de zones de surfaces humaines ou animales ou végétales au moyen d'un plasma froid à pression atmosphérique par production d'une décharge de surface à barrière diélectrique, comportant
- au moins un câble à haute tension (3),
- au moins un câble de masse (4),
- au moins une source de plasma,
- au moins une électrode mise à la masse (1) et
- au moins une électrode à haute tension (2),
**caractérisé en ce que** l'électrode à haute tension (1) est constituée d'un fil souple enrobé dans une couche isolante extensible, et notamment de sorte que l'électrode à haute tension (1) est constituée d'un fil isolant, dans lequel la couche isolante sert de diélectrique pour la décharge à barrière diélectrique à produire, s'adapte simultanément aux contours des zones ou surfaces corporelles à traiter et présente une résistance au claquage d'au moins 2 kV, l'électrode à haute tension (1) étant disposée sur un matériau textile électriquement conducteur servant d'électrode mise à la masse (2).

2. Dispositif selon la revendication 1, **caractérisé en ce que** des polyimides aromatisés sont utilisés comme couche isolante pour l'électrode à haute tension (2).

3. Dispositif selon la revendication 2, **caractérisé en ce que** du poly(4,4'-oxydiphénylène-pyromellitimide) est utilisé comme couche isolante pour l'électrode à haute tension (2).

4. Dispositif selon la revendication 2 ou 3, **caractérisé en ce que** le polyimide aromatisé contient des agents de charge anorganiques.

5. Dispositif selon la revendication 3, **caractérisé en ce que** du Kapton^{®} -CR est utilisé comme couche isolante.

6. Dispositif selon la revendication 1, **caractérisé en ce que** le matériau servant d'électrode mise à la masse (2) est perméable au gaz et souple.

7. Dispositif selon la revendication 6, **caractérisé en ce qu'**une structure de surface textile conductrice sert d'électrode mise à la masse (1), la structure de surface textile étant fabriquée à l'aide des procédé habituels de fabrication de matières textiles.

8. Dispositif selon la revendication 7, **caractérisé en ce que** la structure de surface textile est tissée, tricotée, tressée ou maillée.

9. Dispositif selon la revendication 1, **caractérisé en ce qu'**un matériau conducteur, imperméable au gaz et souple sert d'électrode mise à la masse (1).

10. Dispositif selon l'une des revendications 1 à 9, **caractérisé en ce que** celui-ci présente une alimentation en gaz (6).

11. Dispositif selon la revendication 10, **caractérisé en ce que** celui-ci présente au moins une buse de gaz (5).

12. Dispositif selon l'une des revendications 10 ou 11, **caractérisé en ce que** l'alimentation en gaz (6) peut être interrompue par un dispositif d'obturation, de manière à pouvoir réaliser un traitement dans un volume fermé dans les conditions d'un microclimat sur la surface à traiter.

13. Dispositif selon l'une des revendications 1 à 11, **caractérisé en ce que** l'ensemble d'électrodes de la source de plasma est perméable au gaz et permet ainsi de produite un flux de gaz sur la surface à traiter à travers l'ensemble d'électrodes.

14. Patch, manchette ou pansement pour plaie, destiné(e) à être appliqué(e) sur des zones de peau, et présentant le dispositif selon l'une des revendications 1 à 13.
